# EUROPEAN PATENT APPLICATION

(11) **EP 2 821 500 A1**
(43) Date of publication of application: **07.01.2015**
(21) Application number: 13174786.7
(22) Date of filing: 02.07.2013
(51) Int. Cl.: C12Q 1/26, C12Q 1/54, C12Q 1/60

(54) **Phosphorescence-based hydrogen peroxide assay for the detection of hydrogen peroxide in human serum and water samples**

(71) Applicant: Universität Leipzig, 04109 Leipzig (DE)
(72) Inventor: Kreisig, Thomas, 04179 Leipzig (DE); Prasse, Agneta, 04315 Leipzig (DE); Zscharnack, Kristin, 04299 Leipzig (DE); Züchner, Thole, 69198 Schriesheim (DE)
(74) Representative: Junghans, Claas

(57) **Abstract**

The present invention relates to a method for determining an amount of a peroxide in a sample, wherein the method comprises the steps of:
- providing a sample,
- contacting the sample with a terbium(III) benzene dicarboxylic acid complex, and
- determining the luminescence of the terbium(III) benzene dicarboxylic acid complex.

## Description

The present invention relates to a method for determining an amount of a peroxide, particularly hydrogen peroxide, by determining the luminescence of a lanthanide-ligand complex.

Hydrogen peroxide (H₂O₂) is a highly reactive oxygen species and a strong oxidizer. Hydrogen peroxide is a component of a variety of chemicals industrially applied at large scale, such as suds or disinfectants. Furthermore, hydrogen peroxide is naturally produced as a by-product of several biological processes such as the oxidative metabolization of sugar. Hydrogen peroxide also plays an important role in the immune system, in diseases such as asthma or cancer and as a signalling molecule in the regulation of a variety of biological processes, for example in the regulation of oxidative stress-related states. Therefore, there is a considerable interest in sensitive methods for detection or quantification of hydrogen peroxide, particularly in biological or environmental samples.

A variety of methods for quantifying hydrogen peroxide exist. Among those, luminescence based methods are characterized by high sensitivities. A well-known example for such a luminescence based method is the Europium tetracycline (EuTc) assay, wherein the lanthanide europium is complexed with the antibiotic tetracycline. The luminescence of that complex increases with increasing hydrogen peroxide concentration.

One drawback of this assay is its sensitivity to compounds such as citrate and phosphate in submillimolar and low micromolar concentrations. These compounds increase the fluorescence intensity of the EuTc-complex and interfere with the increase in fluorescence caused by the EuTc-H₂O₂ complex, rendering the assay inaccurate when applied to biological samples.

Thus, it is the objective of the present invention to provide a sensitive and reliable method for the spectroscopic determination of hydrogen peroxide, which is particularly characterized by an increased stability against interfering compounds occurring in biological samples.

According to a first aspect of the invention, a method for determining of an amount of a peroxide is provided, wherein the method comprises the steps of:
- providing a sample,
- contacting the sample with a lanthanide-ligand complex, and
- determining the luminescence of the lanthanide-ligand complex,
   characterized in that the lanthanide-ligand complex is a terbium(III) benzene dicarboxylic acid complex.

The determination of an amount of a peroxide in sense of the present specification particularly refers to the measurement of a concentration of the peroxide, which can easily be converted to the molar / mass amount in a given volume.

The luminescence of the lanthanide-ligand complex changes in presence of the peroxide.

The luminescence may be measured in terms of luminescence intensity or luminescence decay time. Both the intensity and decay time of the lanthanide ligand complex change in presence of the peroxide.

The term "luminescence" in the sense of the present specification refers to the emission of electromagnetic radiation by a substance not resulting from heat, particularly after excitation by electromagnetic radiation. Non-limiting examples for luminescence encompass fluorescence and phosphorescence.

The sample can be any sample, for which the amount of the peroxide needs to be determined. The sample can for example be an environmental sample or a biological sample.

Non-limiting examples for an environmental sample are a sample from waters such as rivers, lakes or oceans, a waste sample, a sewage sample, a soil sample or an air sample.

The peroxide in the sample to be determined may of any origin and may for example originate from biological, geological or industrial processes.

An advantage of the method of the invention is an increased sensitivity of the method for the peroxide. The method provided herein particularly allows to decrease the lower limit of detection (LOD) and the lower limit of quantification (LOQ) to the sub-micromolar range.

Another advantage is an increased insensitivity of the method of the invention against compounds known for their interference in luminescence assays, particularly in lanthanide based assays. Examples for such interfering compounds are citrate and phosphate. The method of the invention compares favourably to state of the art lanthanide based assays such as the EuTc-assay.

Thus, the method of the invention is not disturbed by many salts and other serum components that interfere with the methods known in the art, and is compatible to human serum samples.

As hydrogen peroxide is a byproduct of a number of enzymatic reactions, the method of the invention is also suitable for the detection of these enzymes and their underlying substrates.

The determination of the luminescence may be performed in a suitable container that is permeable to the light emitted by the lanthanide-ligand complex of the invention, and particularly permeable for the light with which the lanthanide-ligand complex can be excited. Examples for such containers include, without being restricted to, microtiter plates, cuvettes, specimen slides and microfluidic chips transparent to light between 200 and 700 nm.

In general, the term peroxide in the sense of the present invention particularly refers to a compound comprising a peroxo group (-O-O-) or a peroxide anion (O₂²⁻).

Likewise, the amount of a compound that decomposes to a peroxide can be determined by the method of the invention, conducting the reaction for example in a protic solvent such as an aqueous solvent. The amount of the peroxide formed by the decomposing reaction can be quantified. An non-limiting example for such a decomposing compound is a compound comprising a superoxide (O₂⁻).

An aqueous solvent in the sense of the sense of the present invention refers to a solvent comprising water, particularly at least 50 % (v/v), 60 % (v/v), 70 % (v/v), 80 % (v/v), 90 % (v/v), 95 % (v/v), or 100 % (v/v) water.

In some embodiments, the peroxide is characterized
- by a general formula 1, 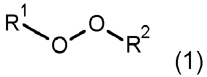 wherein
   R¹ and R² are independently from each other hydrogen, a C₁-C₈ alkyl, a C₁₋₈ cyclic alkyl, a C₅-C₁₀ aryl, a C₁-C₉-heteroaryl, a -C(O)-C₁-C₈ alkyl, a -C(O)-C₁₋₈ cyclic alkyl, a -C(O)-C₅-C₁₀ aryl, a -C(O)-C₁-C₉-heteroaryl, a transition metal or S, wherein S is an acid moiety or a salt thereof,
   or R¹ and R² are a propyl, a butyl or a pentyl forming dioxolane, dioxane or dioxepane ring, wherein the dioxolane, the dioxane or the dioxepane ring may be substituted by a C₁₋₈ alkyl group or may be benzannulated.

A C₁-C₈ alkyl in the context of the present specification signifies a saturated linear or branched hydrocarbon having 1, 2, 3, 4, 5, 6, 7 or 8 carbon atoms, wherein one carbon-carbon bond may be unsaturated and one CH₂ moiety may be exchanged for oxygen (ether bridge). Non-limiting examples for a C₁-C₈ alkyl are methyl, ethyl, 1-propyl, isopropyl, prop-2-enyl, n-butyl, 2-methylpropyl, *tert*-butyl, but-3-enyl, prop-2-inyl, C₂H₅-O-CH₃, but-3-inyl, pentyl, hexyl, heptyl or octyl.

The term aryl in the context of the present specification signifies a cyclic aromatic hydrocarbon. Examples of aryl include, without being restricted to, phenyl and naphthyl. A heteroaryl in the context of the present invention is an aryl that comprises one or several nitrogen, oxygen and/or sulphur atoms. Examples for heteroaryl include, without being restricted to, pyrrole, thiophene, furan, imidazole, pyrazole, thiazole, oxazole, pyridine, pyrimidine, thiazin, quinoline, benzofuran and indole. An aryl or a heteroaryl in the context of the invention additionally may be substituted by one or more alkyl groups.

The term C₁₋₈ cyclic alkyl signifies a cyclic, non-aromatic hydrocarbon having 1, 2, 3, 4, 5, 6, 7 or 8 carbon atoms, wherein one carbon-carbon bond or two carbon-carbon bonds may be unsaturated. Non-limiting examples for a C₁₋₈ cyclic alkyl include cyclopropyl, cylclopropenyl, cyclobutyl, cyclobutenyl, cyclobutadienyl, cyclopentyl, cylcopentenyl, cyclopentdienyl, cylcohexyl, cyclohexenyl, cyclohexadienyl, cylcoheptyl, cycloheptenyl, cylcoheptadienyl, cyclooctyl, cylcooctenyl and cylcoocetadienyl.

In some embodiments, S is selected from -C(O)OH, -S(O₂)OH, -B(OH)₂, a chromate (HCrO₄)⁻, -PO(OH)₂, -NO₃, -N₂OH and SeO(OH),

In some embodiments, the transition metal is selected from Ti^{IV}, V^{V}, Cr^{VI/V}, Mn^{IV}, Co^{III}, Ni^{II}, Zr^{IV}, Nb^{V}, Mo^{VI}, Ru^{II/IV}, Rh^{III}, Pd^{II}, Hf^{IV}, Ta^{V}, W^{VI}, Os^{II/IV}, Ir^{III} and Pₜ^{II}.

In some embodiments, R¹ and R² is the same transition metal.

Examples for such peroxide include, without being restricted to,
- an organic peroxide such as a ether peroxide, a diacylperoxide, a cumolhydroperoxide,
- an inorganic peroxide such as a peroxo borate, a peroxo carbonate,a peroxo chromate, a peroxo cobalt complex, peroxo dicarbonate, a peroxo phosphate, a peroxo diphosphate, a peroxo hyponitrite, a peroxo acyl nitrate, a peroxo dinitrogen(V)oxide, a peroxo sulfuryl halogenide, a peroxo sulphate, a peroxo disulphate
- an inorganic peroxy acid such as peroxymonosulfuric acid, peroxydisulfuric acid, peroxyselenic acid, peroxymonoophosphoric aicd, peroxydiphosphoric acid, peroxynitric acid, peroxymonocarbonic acid, peroxydicarbonic acid, and
- an organic peroxycarboxylic acid such as meta-chloroperoxybenzoic acid and monoperoxyphthalic acid.

In some embodiments, the peroxide is hydrogen peroxide, a radical or a salt thereof, wherein in particular a radical or a salt of hydrogen peroxide decomposes to hydrogen peroxide in an aqueous solvent. Examples for such salts include, without being restricted to, alkali metal salts such as sodium peroxide, earth alkali metal salts such as barium or magnesium peroxide, and transition metal peroxides such as uranyl peroxide.

In some embodiments, the dicarboxylic acid is phthalic acid.

In some embodiments, the peroxide is hydrogen peroxide, and the benzene dicarboxylic acid is phthalic acid.

In some embodiments, the method of the invention is performed in an aqueous solvent.

In some embodiments, the luminescence is determined at a wavelength above 470 nm.

The luminescence may be determined with a photodiode or a photomultiplier, wherein for example the emitted light is filtered by a monochromator that allows only the light with the desired wavelength to pass. Alternatively, the emitted light can be filtered by a cut-off filter that allows only light above a desired wavelength to pass.

In some embodiments, the luminescence is determined at a wavelength of 550±10 nm.

In some embodiments, the luminescence is determined after excitation of the lanthanide-ligand complex of the invention with light characterized by a wavelength of 200 nm to 300 nm.

The lanthanide-ligand complex of the invention may be excited by suitable means such as a lamp, a diode or a laser.

In some embodiments, the luminescence is determined after excitation of the lanthanide-ligand complex with light characterized by a wavelength of 280 nm.

In some embodiments, determining of the luminescence is performed by measuring the luminescence decay time and/or the luminescence intensity of the lanthanide-ligand complex.

In some embodiments, the lanthanide-ligand complex is characterized by a molar ratio of lanthanide to ligand between 3:1 and 2:1, for example 3:1, 2,75:1, 2,5:1, 2,25:1 or 2:1.

In some embodiments, the luminescence is determined at a pH value above 6.

In some embodiments, the luminescence is determined at a pH value between 6.6 and 11.

In some embodiments, the luminescence is determined at a pH between 7 and 11.

In some embodiments, the luminescence is determined at a pH value between 8 and 11.

In some embodiments, the luminescence is determined at pH 8.5.

In some embodiments, the sample is contacted for 2 min, 3 min, 4 min, 5 min, 6 min, 7 min, 8 min, 9 min or 10 min with the lanthanide-ligand complex before the luminescence is determined.

In some embodiments, the sample is selected from the group comprised of blood, sperm, saliva, and an interstitial fluid. In some embodiments, the sample is a body fluid of mammal, particularly a human being. In some embodiments, the sample is a plant or seed material or an extract thereof. In some embodiments, the sample is an environmental sample, for example a freshwater sample, a salt water sample, a waste water sample, a sewage sample, a soil sample or an air sample. In some embodiments, the sample is a cell culture sample.

In some embodiments, the sample is diluted in a suitable solvent system, preferably water, before addition of the lanthanide-ligand-complex of the invention and/or determining the luminescence. Such dilution may be necessary in case of high peroxide concentrations causing too high intensity due to the sensitivity of the method of the invention.

In some embodiments, the hydrogen peroxide to be determined is hydrogen peroxide and is enzymatically generated or consumed.

Such enzymes may use hydrogen peroxide as substrate, for example the enzyme catalase. Such enzymes may also generate hydrogen peroxide in their catalysed reaction, for example oxidases, which use elemental oxygen as electron acceptor.

Determining the amount of hydrogen peroxide generated or consumed by enzymes allows for the determination of the enzymatic activity of these enzymes. Likewise, determining the amount of hydrogen peroxide generated or consumed by enzymes allows for the determination, particularly the quantification, of compounds consumed as substrates or formed as products by the aforementioned enzymes.

In some embodiments, the hydrogen peroxide is generated or consumed by an enzyme selected from glucose oxidase, pyruvate oxidase, lactate oxidase, bilirubin oxidase, alcohol oxidase, sarcosine oxidase, galactose oxidase, amino acid oxidase, monoamine oxidase, cholesterol oxidase, choline oxidase, catalase, superoxide dismutase and urate oxidase.

According to another aspect of the invention, a method for determining a compound is provided, wherein the compound is selected from glucose, galactose, an amino acid, a monoamine, lactate, pyruvate, choline, cholesterol, bilirubin, xanthine, urate, sarcosine, and ethanol, wherein the compound is enzymatically converted, thereby producing or consuming hydrogen peroxide, and the hydrogen peroxide is determined by a method according to the above aspect of the invention. The term "monoamine" in the sense of the present specification refers to compounds characterized by an aromatic ring that is connected to an amino group via an ethylene group, and particularly refers to a neurotransmitter. Such monoamines include, without being restricted to histamine (CAS Nr. 51-45-6), dopamine (CAS Nr. 51-61-6), noradrenaline (CAS Nr. 51-41-2 or 138-65-8), adrenaline (CAS Nr. 51-43-4), serotonine (CAS Nr. 50-67-9), melatonin (CAS Nr. 73-31-4), β-phenylethylamine (CAS Nr. 64-04-0), tyramine (CAS Nr. 51-67-2), tryptamine (CAS Nr. 61-54-1), octopamine (CAS Nr. 104-14-3), 3-iodothyronamine (CAS No. 712349-95-6), thyronamine (CAS Nr. 500-78-7).

In one embodiment, the amount or the concentration of the compound is determined.

According to yet another aspect of the invention, a method for determining the enzymatic activity of an enzyme is provided, wherein the enzyme is selected from the group comprised of glucose oxidase, pyruvate oxidase, lactate oxidase, bilirubin oxidase, alcohol oxidase, sarcosine oxidase, galactose oxidase, amino acid oxidase, monoamine oxidase, choline oxidase, cholesterol oxidase, catalase, superoxide dismutase and urate oxidase. These enzymes consume or form hydrogen peroxide, and the consumption or the formation of the hydrogen peroxide is determined by a method according to the first aspect of the invention.

In some embodiments, the enzyme producing or consuming the hydrogen peroxide is coupled to an antibody. Such enzyme-coupled antibody is particularly useful in an ELISA-assay and may be used as primary antibody for detection of an analyte or as secondary antibody directed against a primary antibody for signal amplification. The amount of the enzyme-coupled antibody can be determined by measurement of the enzymatic activity as described above (yielding an optical signal caused by the luminescence of the lanthanide-ligand-complex of the invention).

According to another aspect of the invention, a method for determining the pH value of a sample is provided, wherein the method comprises the steps of:
- providing a sample, wherein the sample comprises a defined amount of a peroxide, particularly hydrogen peroxide, and
- adding a terbium(III) benzene dicarboxylic acid complex to the sample, and
- determining the luminescence of the terbium(III) benzene dicarboxylic acid complex,

The luminescence of the terbium(III) benzene dicarboxylic acid complex changes with the pH value of the sample.

According to an alternative to the above aspect, a method for determining the pH-value of a sample is provided, wherein the method comprises the steps of.
- providing a sample,
- adding a terbium(III) benzene dicarboxylic acid complex to the sample,
- determining the luminescence of the terbium(III) benzene dicarboxylic acid complex yielding a first luminescence value,
- adding a defined amount of hydrogen peroxide and a terbium(III) benzene dicarboxylic acid complex to the sample, and
- determining the luminescence of the terbium(III) benzene dicarboxylic acid complex yielding a second luminescence value,
wherein the difference between the first and the second luminescence value changes with the pH-value of the sample.

The term sample has the same meaning as described above.

According to another aspect of the invention, a method for determining the amount of an antibody is provided, wherein the antibody is coupled to an enzyme that produces or consumes hydrogen peroxide, the amount of the antibody is determined by the enzymatic activity of the coupled enzymes, and the enzymatic activity is determined by determining the produced or consumed hydrogen peroxide by the method of the invention.

Wherever alternatives for single separable features such as, for example, a certain peroxide or a certain benzene dicarboxylic acid are laid out herein as "embodiments", it is to be understood that such alternatives may be combined freely to form discrete embodiments of the invention disclosed herein. Thus, any of the alternative embodiments for a dicarboxylic acid may be combined with any of the alternative embodiments of a peroxide.

The invention is further characterized, without limitations, by the following examples, from which further features, advantages and embodiments can be derived. The examples are meant to illustrate but not limit the invention.

### Description of the figures

- Fig. 1: shows the phosphorescence intensity of the lanthanide-ligand complex of the invention in presence or absence of hydrogen peroxide in dependence of the lanthanide/ligand molar ratio.
- Fig. 2: shows the phosphorescence intensity of the lanthanide-ligand complex of the invention in presence or absence of hydrogen peroxide in dependence of the pH value.
- Fig. 3: shows a Stern-Volmer-plot of the phosphorescence decrease of the lanthanide-ligand complex of the invention in dependence of the hydrogen peroxide concentration exhibiting the linear range of the assay of the invention and in dependence of the pH value.
- Fig. 4: shows emission spectra of the lanthanide complex of the invention in dependence of the hydrogen peroxide concentration.
- Fig. 5: shows excitation spectra of the lanthanide complex of the invention in dependence of the hydrogen peroxide concentration.
- Fig. 6: shows the signal intensity change over time of the assay of the invention at pH 7.5 (A), pH 8 (B) and pH 8.5 (C).
- Fig. 7: shows the signal response curve of the assay of the invention at different pH values.
- Fig. 8: shows the determination of hydrogen peroxide in human serum and water, wherein the signal intensity of the assay of the invention is plotted versus the hydrogen peroxide concentration.

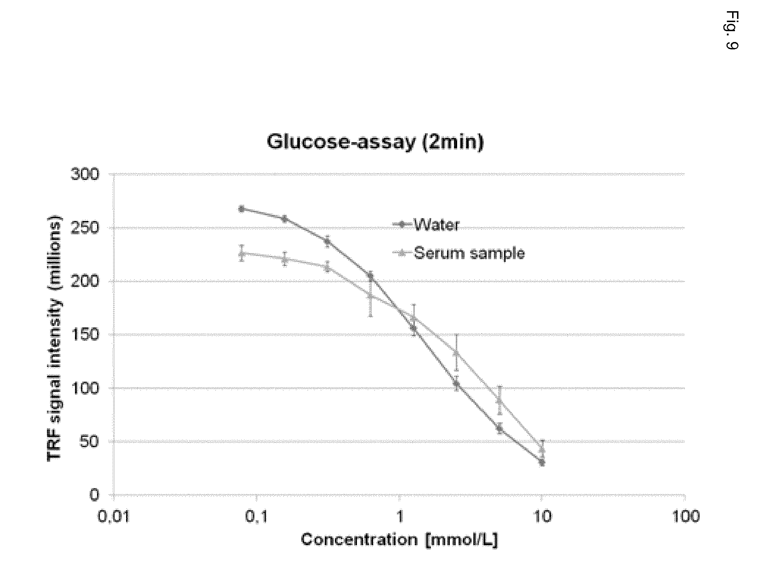

- Fig. 9: shows the determination of glucose in water and human serum after 2 min incubation at room temperature, wherein the signal intensity of the assay of the invention is plotted versus the glucose concentration.
- Fig. 10: shows the determination of glucose in water and human serum after 10 min incubation at room temperature, wherein the signal intensity of the assay of the invention is plotted versus the glucose concentration.

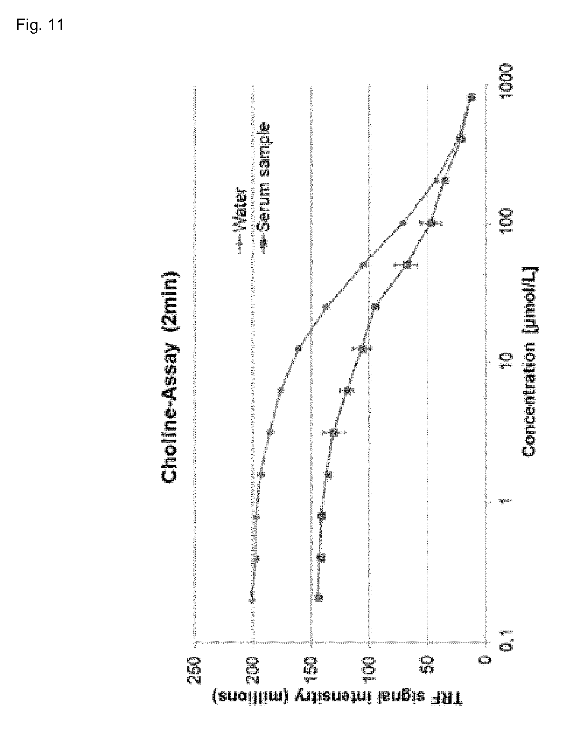

- Fig. 11: shows the determination of choline in water and human serum after 2 min incubation at room temperature, wherein the signal intensity of the assay of the invention is plotted versus the choline concentration.
- Fig. 12: shows the determination of choline in water and human serum after 10 min incubation at room temperature, wherein the signal intensity of the assay of the invention is plotted versus the choline concentration.

### Examples

The assay of the example detects hydrogen peroxide in fluids such as water and serum samples. The assay is based on a phosphorescence signal of phthalic acid in complex with terbium ions, which decreases with increasing concentration of hydrogen peroxide. A certain ratio of phthalic acid to terbium in a suitable buffer is advantageous for an optimal performance of the assay. Suitable buffers are for example HEPES, Tris- or imidazole buffer with a concentration between 50 and 100 mmol/l.

This could be demonstrated for the determination of glucose by using glucose oxidase as a converting enzyme and for choline by using choline oxidase, both for water and serum samples. Other suitable enzymes are those belonging to EC (Enzyme Commission) number 1.11.1. Naturally, all substrates for those enzymes are also potential analytes for this assay.

### Example 1: Hydrogen peroxide determination in aqueous samples

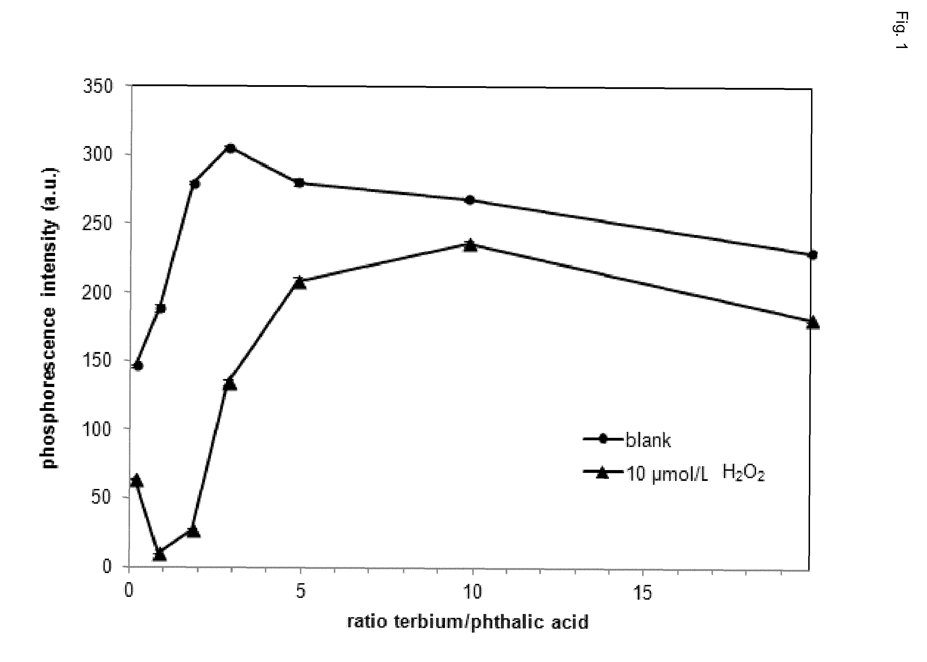

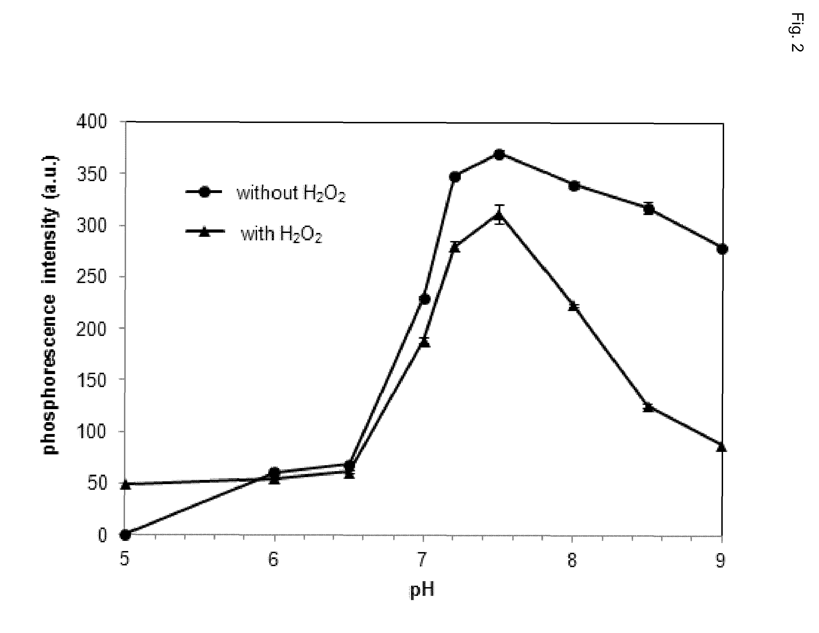

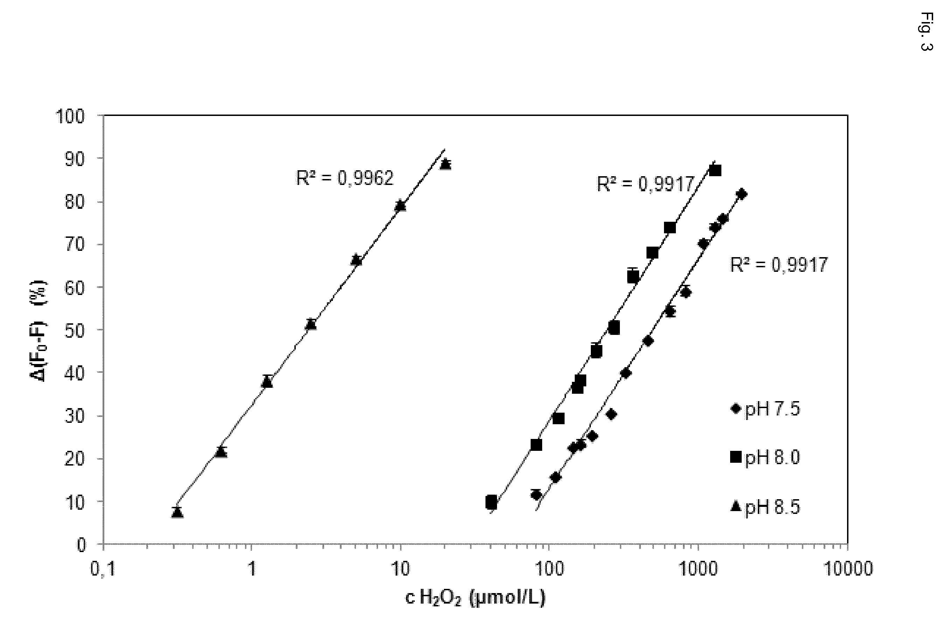

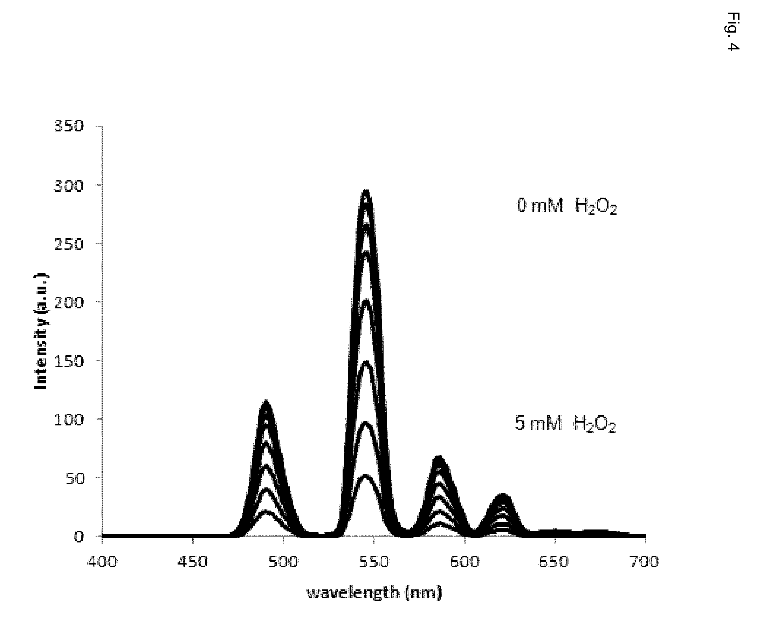

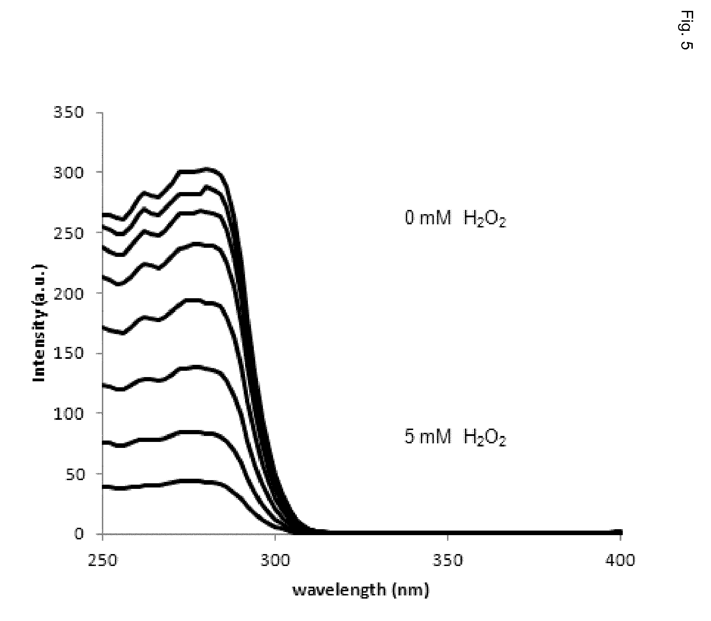

It could be shown that the best terbium/phthalic acid ratio for optimal assay performance in presence or absence of hydrogen peroxide is 3:1 (Fig. 1). However, at a ratio of 2:1 (terbium/phthalic acid) the difference in phosphorescence between presence and absence of hydrogen peroxide is even higher but the limit of detection is worse when compared to a ratio of 3:1. The assay of the invention becomes more sensitive with higher pH (Fig. 2). However, the linear range shifts with increasing pH (Fig. 3). The luminescence of lanthanide-ligand complex (terbium-phthalic acid) can be observed at a wavelength above 470 nm, particularly around 480 nm, around 542 nm, around 580 nm and around 620 nm (Fig. 4) while being excited with a wavelength of 250 nm to 300 nm (Fig. 5).

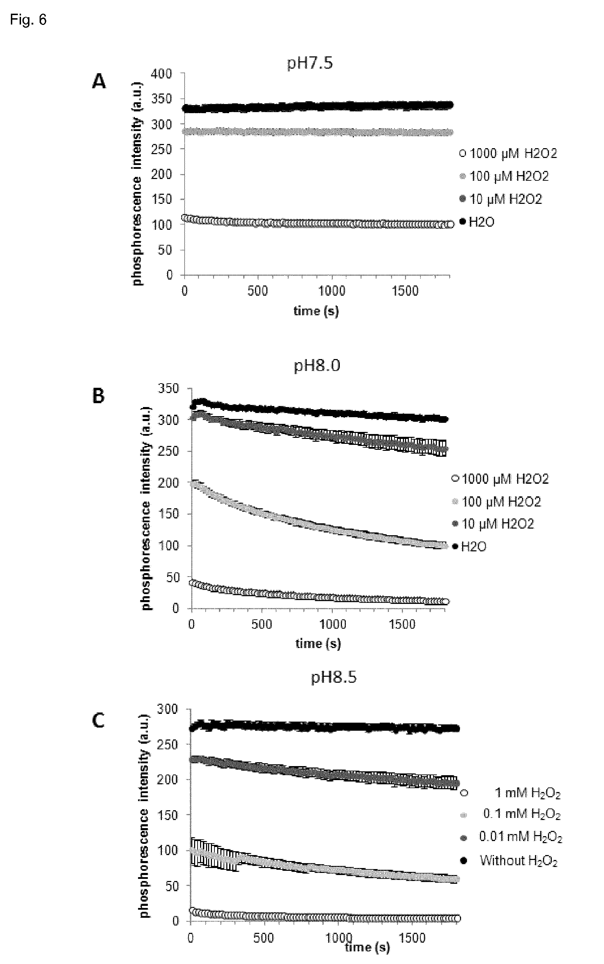

The luminescence signal is relatively stable over a prolonged measurement period, whereby at pH 7.5 virtually no decrease of the signal intensity over a broad range of the hydrogen peroxide concentration can be observed (Fig. 6A). A small but significant decrease of the signal intensity over the time can be observed at higher pH value (Fig. 6 B and C).

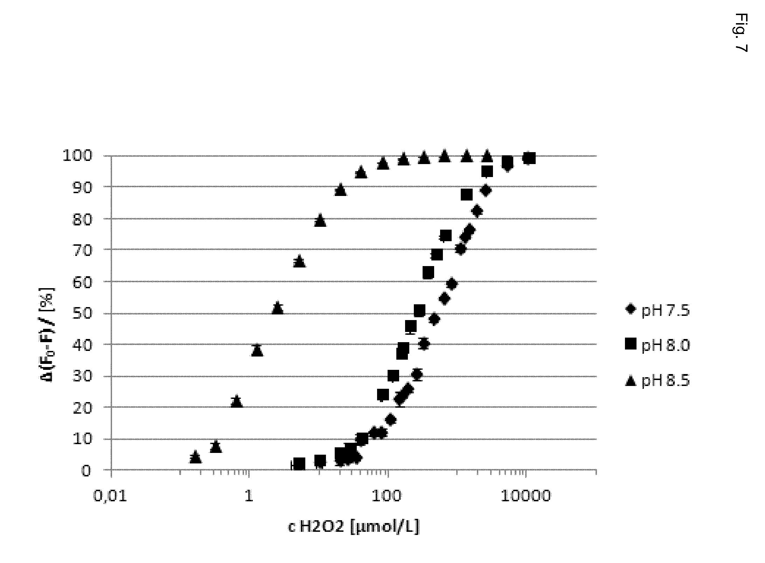

The signal responses of the assay of the invention at different pH values are shown in Fig. 7 and the limits of detection and quantification are listed in the following table 1.

**Table 1**

| pH | limit of detection | limit of quantification |
|---|---|---|
| 7.5 | 40 µmol/l | 108 µmol/l |
| 8.0 | 10 µmol/l | 40 µmol/l |
| 8.5 | 0.156 mol/l | 0.156 µmol/l |

Table 2 shows the recovery rates, intra- and interassay variation coefficient after 3 min incubation.

**Table 2**

| pH | sample | added H₂O₂ (µM) | by Tb₃PS-Assay (µM) | RSD intraassay (%) | RSD interassay (%) | Recovery rate (%) |
|---|---|---|---|---|---|---|
| 7.5 | sample 1 | 1500 | 1508 ± 27.0 | 5.4 | 1.8 | 100.5 |
| | sample 2 | 400 | 380 ± 9.7 | 9.3 | 2.6 | 94.9 |
| | sample 3 | 160 | 148 ± 6.9 | 6.3 | 4.7 | 92.5 |
| | | | | | | |
| 8.0 | sample 1 | 900 | 991 ± 49.3 | 2.3 | 4.9 | 110.1 |
| | sample 2 | 300 | 364 ± 9.5 | 2.2 | 2.6 | 121.3 |
| | sample 3 | 80 | 78 ± 10.5 | 6.9 | 13.4 | 97.3 |

Further, the assay of the invention is characterized by increased stability against a variety of different substances, which frequently occur in biological sample and are known for interfering luminescence assays. None of them interferes with the assay of the invention when physiological concentrations of these substances were used. Table 3 shows a selection of different substances tested on the assay of the invention, wherein the minimal interfering concentration signifies a threshold, under which no interference of the assay can be observed.

**Table 3: Minimal interfering concentration of different substances on the assay**

| compound | minimal interfering concentration | compound | minimal interfering concentration |
|---|---|---|---|
| NaCl | / | Na acetate | > 250 mmol/l; 32 µmol/l - 4 mmol/l |
| NaBr | / | K acetate | / |
| Na₂CO₃ | > 10 mmol/l | Na citrate | / |
| NaHCO₃ | > 10 mmol/l | K citrate | > 40 mmol/l |
| Na₂HPO₄ | > 40 mmol/l | Na lactate | / |
| NaH₂PO₄ | > 40 mmol/l | Na-L-ascorbate | > 20 nmol/l |
| NaF | > 500 mmol/l | BSA | > 0.75% |
| NaJ | > 1mol/l | ethanol | / |
| Na₂SO₄ | > 4 mmol/l | urea | / |
| KCl | / | bilirubin | / |
| KBr | / | glutathione | > 32.6 mmol/l |
| KF | > 40 mmol/l | CsCl | / |
| KJ | > 200 mmol/l | Cs acetate | 1 M |
| K₂SO₄ | / | LiCl | / |
| KHCO₃ | > 80 mmol/l | LiNO₃ | / |
| K₂HPO₄ | > 62.5 mmol/l | Li₂SO₄ | 1 M |
| KH₂PO₄ | > 31.25 mmol/l | MgCl₂ | > 62.5 mmol/l |
| KNO₃ | 1 mol/l; 26 µmol/l-40 nmol/l | MgSO₄ | > 500 mmol/l |
| FeSO₄ | > 80 µmol/l | CaCl₂ | > 125 mmol/l |
| Fe citrate | > 3.2 µmol/l | Ca acetate | > 40 mmol/l |
| CoCl₂ | > 200 µmol/l | CuSO₄ | > 0.1 mmol/l |
| NiCl₂ | > 1mmol/l | CuCl₂ | > 0.1 mmol/l |
| NiSO₄ | > 1 mmol/l | ZnCl₂ | > 1mmol/l |
| BaCl₂ | / | ZnSO₄ | > 10 mmol/l |

### Example 2: Hydrogen peroxide determination in human serum

The assay of the invention is suitable for the detection or quantification of hydrogen peroxide in both aqueous samples and biological samples, in particular in human serum samples.

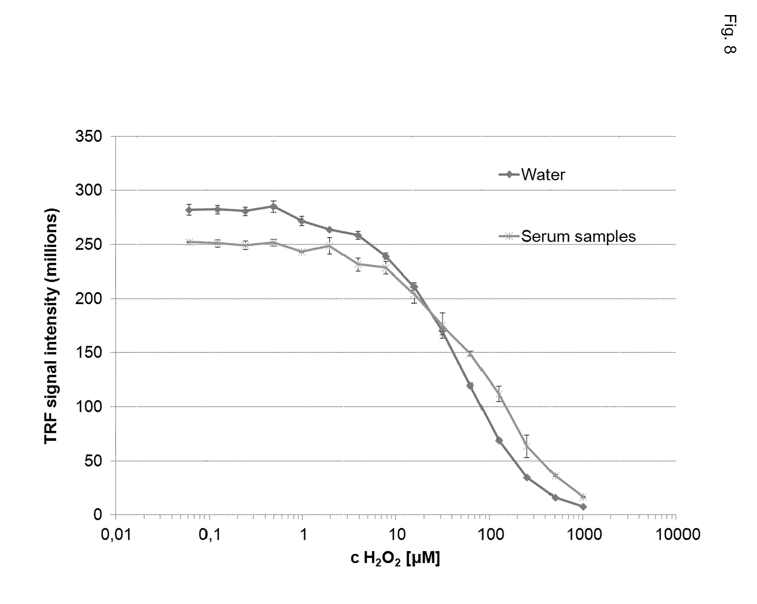

Fig. 8 shows the results of the measurement of hydrogen peroxide in water (dark grey line) and in a serum sample (light grey line).

The determination of hydrogen peroxide was performed as following: a water sample or a serum sample was diluted with water (0.5mL serum plus 9.5mL water) yielding in solution A. Then, 10 µL of solution A and 90 µl of solution B (lanthanide complex, 2.33 mmol/L terbium, 0.77 mmol/L phthalic acid in 80 mmol/L HEPES buffer, pH 8.0) were added to a microtiter plate, mixed and incubated at room temperature for 3 minutes. After incubation the luminescence (phosphorescence) of the lanthanide ligand complex of the invention was measured at an emission wavelength 550 nm after excitation at 280 nm with a (time resolved) fluorescence plate reader.

### Example 3: Glucose determination in human serum

The assay of the invention can also be used for the enzymatic determination of substances which are converted with or to hydrogen peroxide, such as glucose that is converted by the glucose oxidase to glucono lactone and hydrogen peroxide.

The determination of glucose was performed as following: 0.5 ml serum sample or water sample was diluted with 9.5 ml assay buffer yielding in solution A. Then, 10 µL of solution A, 85 µl of solution B (lanthanide complex, 2.33 mmol/L terbium, 0.77 mmol/L phthalic acid in 80 mmol/L HEPES buffer, pH 8.0) and 5 µL of glucose oxidase solution (0.1 units in HEPES buffer, pH 8.0, 100 mmol/L, wherein one unit will oxidize 1.0 µmol of β-D-glucose to D-gluconolactone and H₂O₂ per min at pH 5.1 at 35 °C, equivalent to an O₂ uptake of 22.4 µL per min) were added to a microtiter plate, mixed and incubated at room temperature for 2 minutes. After incubation the luminescence (phosphorescence) of the lanthanide ligand complex of the invention was measured at an emission wavelength 550 nm after excitation at 280 nm with a (time resolved) fluorescence plate reader. If the reaction mixture is saturated with oxygen, the activity may increase by up to 100%.

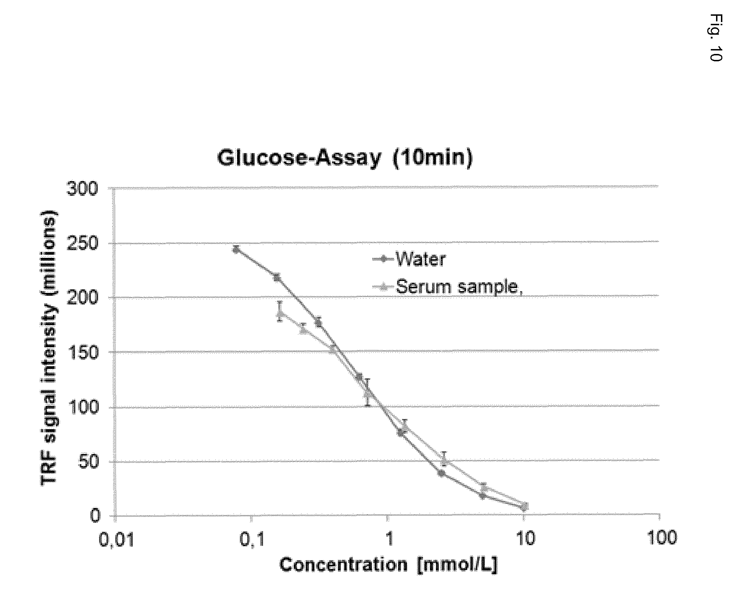

A fourfold improvement in sensitivity can be achieved by increasing the incubation time from 2 minutes to 10 minutes (fig. 10).

### Example 4: Choline determination in human serum

Another application of the assay of the invention is the enzymatic determination of choline, which is converted by the choline oxidase to glycine betaine aldehyde and hydrogen peroxide.

The determination of choline was performed as following: 0.5 ml serum sample or water sample was diluted with 9.5 ml assay buffer yielding in solution A. Then, 10 µL of solution A, 45 µl of solution B (lanthanide complex, 2.33 mmol/L terbium, 0,77 mmol/L phthalic acid in 80 mmol/L HEPES buffer, pH 8.0) and 45 µL of choline oxidase solution (0.9 units in HEPES buffer, pH 8.0, 100 mmol/L, wherein one unit will form 1 µmol of H₂O₂ with oxidation of 1 µmol of choline to betaine aldehyde per min at pH 8.0 at 37 °C) were added to a microtiter plate, mixed and incubated at room temperature for 2 minutes. After incubation the luminescence (phosphorescence) of the lanthanide ligand complex of the invention was measured at an emission wavelength 550 nm after excitation at 280 nm with a (time resolved) fluorescence plate reader. Note, that during the conversion of choline to betaine by choline oxidase, 2 µmol of H₂O₂ are produced for every µmol of choline.

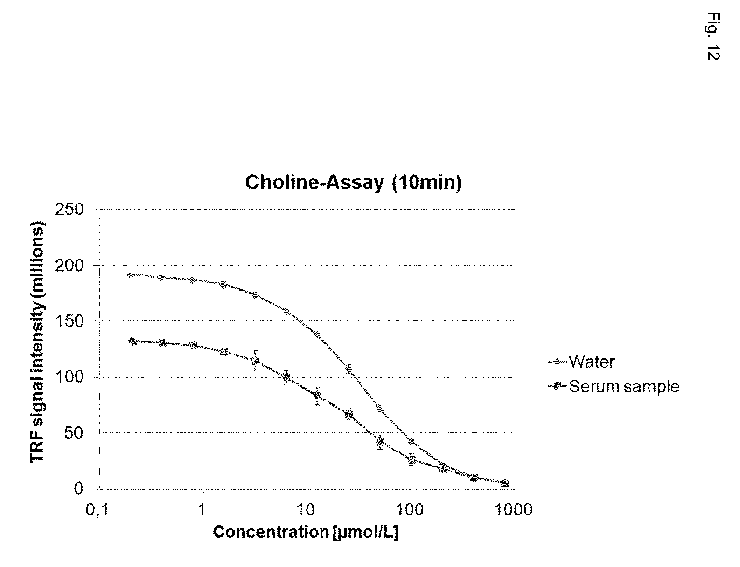

Here, an even twenty-fivefold improvement in sensitivity can be achieved by increasing the incubation time from 2 minutes to 10 minutes (fig. 12).

## Claims

1. A method for determining an amount of a peroxide, in a sample, comprising the steps of :
- providing a sample,
- contacting said sample with a lanthanide-ligand complex, and
- determining the luminescence of said lanthanide-ligand complex,
**characterized in that** said lanthanide-ligand complex is a terbium(III) benzene dicarboxylic acid complex.

2. The method according to claim 1, wherein said peroxide is hydrogen peroxide.

3. The method according to claim 1 or 2, wherein said benzene dicarboxylic acid is phthalic acid.

4. The method according to any one of the preceding claims, wherein said luminescence is determined at a wavelength above 470 nm, preferably at a wavelength of 550±10 nm.

5. The method according to any one of the preceding claims, wherein said luminescence is determined after excitation of said lanthanide-ligand complex with light **characterized by** a wavelength of 200 nm to 300 nm, preferably by a wavelength of 280 nm.

6. The method according to any one of the preceding claims, wherein determining the luminescence is performed by measuring the luminescence decay time and/or the luminescence intensity of said lanthanide-ligand complex.

7. The method according to any one of the preceding claims, wherein said lanthanide-ligand complex is **characterized by** a molar ratio of lanthanide to ligand between 3:1 and 2:1.

8. The method according to any one of the preceding claims, wherein said luminescence is determined at a pH-value between 6.6 and 11

9. The method according to any one of the preceding claims, wherein said sample is contacted for 2 min, 3 min, 4 min, 5 min, 6 min, 7 min, 8 min, 9 min or 10 min with said lanthanide-ligand complex before said luminescence is determined.

10. The method according to any one of the preceding claims, wherein said sample is selected from blood, sperm, saliva, an interstitial fluid or another body fluid, plant or seed material or another biological sample or an environmental sample.

11. The method according to any one of claims 2 to 10, wherein said hydrogen peroxide is enzymatically generated or consumed.

12. The method according to claim 11, wherein said hydrogen peroxide is generated or consumed by an enzyme selected from glucose oxidase, pyruvate oxidase, lactate oxidase, bilirubin oxidase, alcohol oxidase, sarcosine oxidase, galactose oxidase, amino acid oxidase, monoamine oxidase, cholesterol oxidase, choline oxidase, catalase, superoxide dismutase and urate oxidase.

13. A method for determining an amount of a compound selected from glucose, galactose, an amino acid, a monoamine, lactate, pyruvate, choline, cholesterol, bilirubin, xanthine, urate, sarcosine, and ethanol, wherein said compound is enzymatically converted, thereby producing or consuming hydrogen peroxide, and said hydrogen peroxide is determined by a method according to any one of claims 1 to 12.

14. A method for determining the enzymatic activity of an enzyme consuming or forming hydrogen peroxide selected from the group comprised of glucose oxidase, pyruvate oxidase, lactate oxidase, bilirubin oxidase, alcohol oxidase, sarcosine oxidase, galactose oxidase, amino acid oxidase, monoamine oxidase, choline oxidase, cholesterol oxidase, catalase, superoxide dismutase and urate oxidase, wherein hydrogen peroxide is determined by a method according to any one of claims 1 to 12.
